(19) Europäisches Patentamt
European Patent Office
Office européen des brevets

(11) EP 0 789 080 B1

(12) **EUROPÄISCHE PATENTSCHRIFT**

(45) Veröffentlichungstag und Bekanntmachung des Hinweises auf die Patenterteilung:
**16.01.2002 Patentblatt 2002/03**

(51) Int Cl.[7]: **C12P 7/56**, C07D 319/12

(21) Anmeldenummer: **97250027.6**

(22) Anmeldetag: **07.02.1997**

(54) **Verfahren zur Herstellung von organischen Aminiumlactaten und deren Verwendung zur Herstellung von Dilactid**

Process for the preparation of organic aminium lactates and their use in the preparation of dilactide

Procédé de préparation de lactates d'aminium organiques et leur utilisation pour le préparation de dilactide

(84) Benannte Vertragsstaaten:
**AT BE CH DE DK FI FR IT LI LU NL SE**

(30) Priorität: **08.02.1996 DE 19606303**
**04.07.1996 DE 19628519**

(43) Veröffentlichungstag der Anmeldung:
**13.08.1997 Patentblatt 1997/33**

(73) Patentinhaber:
- **Gesellschaft für ökologische Technologie und Systemanalyse e.V.
10178 Berlin (DE)**
- **Institut für Agrartechnik Bornim e.V. ATB
14469 Potsdam-Bornim (DE)**

(72) Erfinder:
- **Kamm, Birgit, Dr.
14513 Teltow (DE)**
- **Kamm, Michael
14513 Teltow (DE)**
- **Teichmann, Bodo, Prof. Dr.
13125 Berlin (DE)**
- **Richter, Klaus, Dr.
04209 Leipzig (DE)**
- **Soyez, Konrad, Dr.
10178 Berlin (DE)**
- **Reimann, Winfried, Dr.
14473 Potsdam (DE)**

(74) Vertreter: **Ziebig, Marlene, Dr. Dipl.-Chem. et al
Patentanwälte Gulde Hengelhaupt Ziebig
Robert-Rössle-Strasse 10
13125 Berlin (DE)**

(56) Entgegenhaltungen:
**WO-A-93/06226** **FR-A- 2 555 200**
**NL-C- 2 169**

- **JOURNAL OF THE AMERICAN CHEMICAL SOCIETY, Bd. 56, Nr. 8, 4.August 1934, Seiten 1759-1760, XP002056785 POLLARD C.B. ET AL.: "Derivatives of piperazine. II. Utilization in identification of fatty acid."**
- **CHEMICAL ABSTRACTS, vol. 80, no. 14, 8.April 1974 Columbus, Ohio, US; abstract no. 71985, TOMIKAZU O.: "Linear coployamide used for textiles and having desirable moisture retaining properties." Seite 67; Spalte 1; XP002056786 & JP 47 044 995 B (TEIJIN LTD.) 13.November 1972**
- **CHEMICAL ABSTRACTS, vol. 107, no. 5, 3.August 1987 Columbus, Ohio, US; abstract no. 39822, CARRERAS GINJAUME E.: "Process for the preparation of imidazole lactate." Seite 703; Spalte 2; XP002056787 & ES 540 741 A (CARRERAS GINJAUME, EUSEBIO) 16.Mai 1986**

EP 0 789 080 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft ein Verfahren zur Herstellung von organischen Aminiumlactaten oder Diaminiumdilactaten bei der Fermentation von kohlenhydrathaltigen Substraten zur Erzeugung von Milchsäure, wobei die Neutralisation der Milchsäure mit geeigneten Aminen erfolgt. Die entstehenden neuen, gut kristallisierenden Aminiumlactate heterocyclischer sekundärer oder tertiärer Amine oder Diamine sind stabil und können als direkte Ausgangsstoffe zur Herstellung von Dilactid dienen.
Es werden bei der Fermentation sowohl racemische als auch optisch aktive Salze oder nicht drehende optisch inaktive Salze in Abhängigkeit vom eingesetzten Mikroorganismus isoliert.

In NL 2 169 C ist ein Verfahren zur Milchsäuregärung unter Verwendung von Ammoniak, Ammoniumverbindungen oder Urotropin als Neutralisationsmittel beschrieben. Vorzugsweise wird Ammoniumcarbonat benutzt. Das erwähnte Urotropin (chemisch Hexamethylentetramin) ist ein Additionsprodukt aus Ammoniak und Formaldehyd, das in wäßriger Lösung in Gegenwart von Säuren instabil ist und wieder in seine Bestandteile gespalten wird. Seine Zugabe stellt eine Spezialvariante der Neutralisation mit Ammoniak dar. Da Urotropin unter Fermentationsbedingungen zerfällt, kann es keine stabilen Aminiumsalze bilden.

Auch das aus FR 2 555 200 A bekannte Verfahren zur Herstellung von Milchsäure aus Molke benutzt eine wäßrige Ammoniaklösung zur pH-Regelung. Stabile Salze können nicht entstehen.

Weiterhin ist in WO 93/06226 A ein mehrstufiges Verfahren als eine Variante der extraktiven Abtrennung von Milchsäure aus Fermentationsmedien beschrieben. Extraktionsmittel sind wasserunlösliche Ionenaustauscherharze, die u.a. tertiäre Amino-Endgruppen tragen. Die als wirksame Komponenten erwähnten Polyvinylpyridin-Verbindungen sind polymere Makromoleküle, die auf ihrer Oberfläche mit aminischen Endgruppen substituiert sind. Sie kommen mit dem Fermentationmedium, das zellfrei sein muß, nur in einem Außenkreislauf in Kontakt, d.h. sie gelangen nicht in den Fermentor. Bei der bakteriellen Fermentation wird dazu die Biomasse vorher über eine Ultrafiltrationsmembran vom Medium abgetrennt und im Fermentor zurückgehalten. Der Fermentationsprozeß verläuft in zwei Phasen. Zunächst erfolgt eine gewöhnliche diskontinuierliche Fermentation, bei der die pH-Regulierung mit Natronlauge erfolgt. Ab einem bestimmten Zeitpunkt wird dann durch Abschaltung der pH-Regelung eine moderate Absenkung des pH-Wertes erreicht, wobei freie Milchsäure entsteht, die dann nach Einschalten des Kreislaufes zusammen mit der im weiteren Prozeßverlauf gebildeten freien Milchsäure mit Hilfe der beschriebenen Ionenaustauscherharze extrahiert wird. In dieser Phase ist keine pH-Regelung erforderlich, da die pH-senkende Milchsäure sofort aus dem Medium entfernt wird. Die auf den festen Austauscherharzen adsorbierte Milchsäure muß dann noch unter Zugabe von Elutionsmitteln, wie z.B. Methanol, von den Säulen heruntergewaschen werden, wobei wäßrige Lösungen der freien Milchsäure entstehen.

Aufgabe der Erfindung war es, ein möglichst einfaches und hochproduktives Fermentationsverfahren bereitzustellen, mit dem die entstehende Milchsäure im Fermentationsmedium in gut kristallisierbare Salze überführt werden kann, die dann direkt als Ausgangsstoffe für die Dilactidsynthese dienen können.

Es hat sich gezeigt, daß heterocyclische sekundäre oder tertiäre Amine oder Diamine, wie z.B. Piperidin, Pyrrolidin, Imidazol, 4-Dimethylaminopyridin oder deren Derivate, und auch Aminozucker, wie z.B. D-Glucosamin, mit Milchsäure, stabile, gut kristallisierende Salze bilden können.

Die genannten Amine werden zur Isolierung von Milchsäure aus Fermentationsmedien eingesetzt, wodurch der gesamte Fermentationsprozeß wesentlich effektiviert wird.

Außerdem betrifft die Erfindung neue Diaminiumdilactate der allgemeinen Formel V gemäß Anspruch 7, insbesondere D,D-; L,L-; D,L- und meso-Piperazonium- bzw. Alkylpiperazoniumdilactate.

Die neuen Diaminiumdilactate besitzen die allgemeine Formel V:

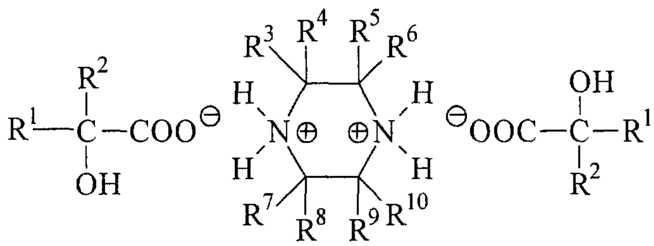

(V)

in der

$R^1$ und $R^2$ unabhängig voneinander Wasserstoff oder $CH_3$ bedeuten,

und

$R^3$ - $R^{10}$ unabhängig voneinander Wasserstoff, verzweigtes oder unverzweigtes $C_1$ - $C_{10}$ -Alkyl, $C_1$ - $C_{10}$ -Alkoxy, Aralkyl mit 1 bis 10 aliphatischen Alkylgruppen zwischen Aromat und Piperazinkörper,
Aryl und durch Alkyl-, Alkoxy-,Aryl-, Halogen-, Nitro-, Cyano-, Sulfonyl- Reste substituiertes Aryl, Allyl, Halogen, Nitril, Isonitril sowie Teile eines ankondensierten aliphatischen, aromatischen oder heterocyclischen Systems bedeuten,

ausgenommen racemisches 1,4-Piperazoniumdilactat.

Erfindungsgemäß wurde auch Imidazoliumlactat als D- und L-Form gemäß Anspruch 8 gewonnen, ebenso wie racemisches 4-Dimethylaminopyridiniumlactat bzw. dessen L-oder D-Form.

Die erfindungsgemäßen Piperazoniumsalze der Formel V, das Imidazoliumlactat oder das 4-Dimethylaminopyridiniumlactat können zur Herstellung von 1,4-Dioxan-2,5-dionen verwendet werden. Insbesondere aufgrund ihrer optischen Aktivität sind sie als Synthone für stereospezifische, enatiomere Synthesen, beispielsweise zur Herstellung optisch aktiver D,D-, L,L-Diester und optisch inaktiver meso- und racemischer Diester sehr gut geeignet. Darüber hinaus besitzen die Verbindungen V auch biologische Aktivität.

Tabelle 1 zeigt eine Auswahl von erfindungsgemäßen Aminiumsalzen und ihre Eigenschaften.

Gegenstand der Erfindung ist auch ein hochproduktives Fermentationsverfahren gemäß den Ansprüchen 1 - 6, bei dem die entstandene Milchsäure durch heterocyclische Amine oder Aminozucker, die keine toxische oder stark hemmende Wirkung auf die Aktivität der Milchsäurebakterien ausüben, im Fermentationsmedium in gut kristallisierbare Salze überführt wird, die dann als direkte Ausgangsstoffe für die Dilactidsynthese hervorragend geeignet sind.

Dilactide sind die Ausgangsstoffe für abbaubare Polymere mit sehr vielfältigen Anwendungsmöglichkeiten als Verpackungsmaterial oder Spezialwerkstoffe. Zur Erzeugung von Dilactid wurde bisher Milchsäure verwendet, die man als L(+)-Lactat bzw. D(-)-Lactat durch Vergärung von kohlenhydrathaltigen Medien erhält. Dabei wird die Milchsäure in freier Form oder derivatisiert als Ester katalytisch in das dimere Lactid umgesetzt. Dieser Prozeß ist sehr energieintensiv und durch große Produktverluste gekennzeichnet.

Milchsäure wird mit den bekannten Verfahren gewöhnlich als eine 80-90%ige wäßrige Lösung produziert. Diese Verfahrensweise ist aufwendig, da man wegen der auf die Milchsäurebakterien wirkenden Inhibierung durch die gebildete Milchsäure bei der Fermentation ein Neutralisationsmittel (Calciumcarbonat) einsetzen muß. Das dadurch gebildete Lactat muß dann -in einer nachfolgenden Stufe wieder in die freie Säure umgewandelt werden, wozu man konzentrierte Schwefelsäure benötigt. Als schwer verwertbares Abfallprodukt fällt Sulfatschlamm an. In einigen Fällen wird dieser Nachteil dadurch überwunden, daß Natron-oder Kalilauge bzw. Ammoniak zur Neutralisation des Fermentationsmediums verwendet wird und die Freisetzung der Milchsäure in einer nachgeordneten Verfahrensstufe durch Ionenaustauscher (WO 93/06226 und EP 517 242) oder Elektrodialyse (EP 657 542 und PL 161 321) erfolgt.

Mit anderen Verfahren gewinnt man auch Lactate anorganischer Basen, wie z.B. Magnesiumlactat (CZ 279 449) oder Natriumlactat (CN 1 063 485).

Die Herstellung von Milchsäureestern von $C_1$-$C_4$-Alkanolen als Ausgangsprodukte für die Dilactidsynthese wird in EP-A-614 983 und DE 43 41 770 A1 beschrieben. DE 43 41 770 A1 offenbart ein Fermentationsverfahren zur Herstellung von Milchsäureestern, bei dem die Milchsäure zunächst mit Calciumcarbonat neutralisiert und dann durch Zusatz von Ammoniak Ammoniumlactat erhalten wird, das entweder direkt mit Alkohol verestert wird oder mit einem tertiären Alkylamin zur Bildung einer Trialkyl-Ammoniumlactatlösung versetzt wird, die dann ihrerseits mit dem Alkohol verestert wird. Auch dieses Verfahren ist also durch eine mehrfache Konvertierung der Milchsäure gekennzeichnet, bis ein Milchsäureester erhalten wird, der dann in das dimere Lactid umgesetzt werden kann.

Demgegenüber ist das erfindungsgemäße Verfahren zur Herstellung von organischen Aminiumlactaten oder Diaminiumlactaten durch Fermentation von kohlenhydrathaltigen Substraten dadurch gekennzeichnet, daß die Milchsäure in einem Schritt neutralisiert und gleichzeitig in ein direktes Ausgansprodukt für die Dilactidsynthese, nämlich in ein gut kristallisierbares Aminiumlactat oder Diaminiumdilactat, umgewandelt wird. Das erfindungsgemäße Verfahren gemäß Anspruch 1 besteht darin, daß dem Fermentationsmedium zur Neutralisierung der entstehenden Milchsäure ein Amin oder Diamin zugegeben wird, das für die eingesetzten Mikroorganismen nicht toxisch ist und auch deren Aktivität nicht hemmt, so daß am Ende der Fermentation der pH-Wert der Flüssigkeit im Bereich von 4,5 bis 13,5 liegt, vorzugsweise im Bereich von 5,0 bis 8,0. Die Fermentationsbrühe wird dann in an sich üblicher Weise, bevorzugt mit Hilfe von Ultrafiltration, Elektrodialyse und Umkehrosmose vorgereinigt und das Aluminiumlactat darin aufkonzentriert, wobei die bei der Elektrodialyse anfallende Fraktion von nichtionischen Nährstoffen in die Fermentationsstufe und die in der Umkehrosmose abgereicherte Phase in die Elektrodialyse zurückgeleitet werden kann. Danach wird das so erhaltene Aminiumlactat-Konzentrat eingeengt, vorzugsweise im Vakuum, und mit einem Lösungsmittel versetzt, wobei gleichzeitig die restlichen, noch im Gemisch enthaltenen Nährlösungsbestandteile (z.B. Pepton) als Niederschlag ausfallen und abfiltriert werden können sowie in den Fermentationsprozeß zurückgeführt werden können. Das Aminiumlactat verbleibt in der Lösung, aus der man es in kristalliner Form gewinnt.

Es hat sich gezeigt, daß erfindungsgemäß solche Amine und Diamine in Frage kommen, die nicht toxisch für die eingesetzten Mikroorganismen sind und auch deren Aktivität nicht hemmen und mit Milchsäure gut kristallisierbare Salze bilden. Insbesondere kommen als Amine und Diamine heterocyclische sekundäre Amine oder Diamine, wie z. B. Piperazin, Piperidin oder Pyrrolidin bzw. deren Salze oder Hydrate oder Derivate davon in Frage. Aber auch heterocyclische Stickstoffverbindungen mit Doppelbindungen innerhalb des Ringes oder deren Derivate, wie z.B. Imidazol oder 4-Dimethylaminopyridin, sind für das erfindungsgemäße Fermentationsverfahren geeignet, wobei diese Heterocyclen auch mit anderen Ringsystemen kondensiert oder substituiert sein können. Daneben hat sich gezeigt, daß auch Aminozucker, z.B. D-Glucosamin, erfindungsgemäß im Fermentationsverfahren einsetzbar sind.

Eine bevorzugte Variante des erfindungsgemäßen Verfahrens besteht darin, daß ein heterocyclisches sekundäres Amin, z.B. Piperazin, in konzentrierter wäßriger Lösung dem Fermentationsmedium bei der pH-Wert-Regelung als Neutralisationsmittel zugesetzt wird. Die Zudosierung kann als freie Base, jedoch auch in fester Form als Pulver, Granulat etc. oder in nichtwäßriger Lösung erfolgen, aber auch als Salz (Carbonat, Hydrogencarbonat) oder an geeignete Ionenaustauscher gebunden.

Außerdem besteht die Möglichkeit, die Neutralisierung der Milchsäure außerhalb des Fermentors vorzunehmen, z.B. im Ausgangsstrom einer mit der Fermentation gekoppelten Elektrodialyse- oder Ionenaustauscher-Stufe. Für diesen Fall ist es nicht unbedingt erforderlich, daß ein für die Mikroorganismen nichttoxisches Amin eingesetzt wird, da die Biomasse in der Regel bereits in einer davorliegenden Ultrafiltrationsstufe abgetrennt und gegebenenfalls in den Fermentor zurückgeführt wurde.

Das erfindungsgemäße Verfahren zeichnet sich gegenüber bekannten Prozessen dadurch aus, daß keine größeren Mengen an festen Nebenprodukten oder Abfällen entstehen und daß alle Stoffausgangsströme vorwiegend in flüssiger Form im Gesamtprozeß wieder eingesetzt werden können. Auch das organische Amin kann bei der sich an die Fermentation anschließenden Cyclisierung des Aminiumlactates oder Diaminiumlactates zum Dilactid unzersetzt zurückgewonnen und wieder im Fermentationsprozeß eingesetzt werden.

Ein Verfahrensschema für eine Vorzugsvariante des erfindungsgemäßen Fermentationsverfahrens zeigt die Abbildung 2.

In einer bevorzugten Ausführungsvariante des erfindungsgemäßen Fermentationsverfahrens wird ein Medium, das vergärbare Zucker in Mengen von 50g/l bis 150g/l und weitere Wuchsstoffe (z.B. Hefeextrakt, Pepton) und Salze enthält, mit jeweils einem Bakterienstamm der Gattungen Lactobacillus oder Streptococcus bzw. mit ihren Gemischen oder mit einem Pilzstamm der Gattung Rhizopus in bekannter Weise bei Temperaturen von 25 - 60°C kultiviert.

Der Hauptteil der im Prozeßverlauf gebildeteten Milchsäure wird im Fermentationsmedium durch die bedarfsgerechte Zugabe einer konzentrierten wäßrigen Aminlösung neutralisiert, wobei Aminiumlactat entsteht und der pH-Wert im Bereich von 4,0-8,0 vorzugsweise bei 5,0-6,5 konstant gehalten wird. Nach 10 bis 24 h, wenn der gesamte Zucker umgesetzt ist, kann der Fermentationsprozeß abgebrochen werden. Der pH-Wert wird dann durch Zugabe des organischen Amins auf einen Wert von 4,5 bis 13,5 vorzugsweise von 5,0 bis 8,0 eingestellt.

Die Fermentationsbrühe wird anschließend über eine Ultrafiltrationszelle geleitet, wobei die gesamte Biomasse und alle anderen Feststoffanteile abgetrennt und entweder in den Fermentor zurückgeführt oder über eine Filterpresse aus dem Prozeß entfernt und als Silierhilfs- oder Futtermittel eingesetzt werden können.

Das klare Filtrat gelangt dann in eine Elektrodialysekammer, wo eine Anreicherung aller ionischen Bestandteile, insbesondere des organischen Aminiumlactates, erfolgt. Die wäßrige Restphase, die alle nichtionischen Nährlösungsbestandteile und einen geringen Teil Aminiumlactat enthält, kann ebenfalls in die Fermentationsstufe zurückgeleitet werden.

Eine weitere Aufkonzentrierung des organischen Aminiumlactates im Elektrodialysat erfolgt durch Umkehrosmose, gegebenenfalls gekoppelt mit einer fraktionierten Kristallisation des Produktes. Dabei lassen sich auch anorganische Ionen aus der Aminiumlactatlösung abtrennen. Der produktarme Teilstrom aus der Umkehrosmose wird in die Elektrodialyse-Stufe recyclisiert. Dieser Vorgang ist gegebenenfalls zu wiederholen.

Die gesammelten Aminiumlactat-Konzentrate werden vorzugsweise unter Vakuum entwässert, in einem geeigneten Lösungsmittel, wie z.B. Alkohol, Ethylenglycolmono-bzw. Ethylenglycoldialkylether ($C_1$ bis $C_5$- Glycolether), Acetonitril, Essigsäurealkylester, Dioxan, DMSO oder DMF gegebenenfalls unter Erwärmen gelöst und von ausfallenden Niederschlägen durch Filtration befreit. Aus den klaren Lösungen wird dann das organische Aminiumlactat in kristalliner Form erhalten.

Das erfindungsgemäße Fermentationsverfahren zeichnet sich gegenüber bekannten Prozessen durch den Wegfall von umweltbelastenden festen Abfällen und durch nur schwachbelastete Abwässer aus. Es sind keine mehrfachen Umsalzungen notwendig und Milchsäureverluste durch voluminöse Niederschläge und unkontrollierte Polymerisation bei der Produktaufkonzentrierung entfallen. Die mengenmäßig geringen Filtrationsrückstände lassen sich zusammen als Silierhilfs- oder Futtermittel verwenden. Auch eine Entsorgung durch Verbrennung ist möglich. Beide im Prozeßschema angeführten Feststoffabtrennungen können zeitlich versetzt in der gleichen Vorrichtung durchgeführt

werden. Zur Entlastung des Stoffkreislaufes gelangt das Elektrodialysat gegebenenfalls auch direkt in die Verdampfung. Der Fermentationsprozeß kann diskontinuierlich, semikontinuierlich oder kontinuierlich ablaufen.

Mit Hilfe des erfindungsgemäßen Fermentationsverfahrens und durch Anwendung geeigneter Stämme gelingt es problemlos, optisch reine Aminiumlactate bzw. Diamoniumdilactate oder definierte Gemische ihrer D-und L-Formen unter völligem Ausschluß von oligomeren Milchsäureverbindungen zu isolieren, die bei der Aufkonzentrierung von Milchsäure zwangsläufig entstehen und bei der Dilactid-Synthese hinderlich sein können. Damit steht für eine nachfolgende Dilactid-Synthese ein genau definierter Ausgangsstoff zur Verfügung.

Anschließend soll die Erfindung an Ausführungsbeispielen näher erläutert werden, ohne sie jedoch darauf zu beschränken.

## Ausführungsbeispiele

### Beispiel 1

Eine Lösung von 5 g Pepton, 5 g Hefeextrakt, 2,0 g Ammoniumphosphat, 1 g Kaliumhydrogenphosphat in 1 1 Leitungswasser und das gleiche Volumen einer 17%igen Glucoselösung werden getrennt sterilisiert und dann zusammen in einen Rührfermentor gegeben. Der pH-Wert dieses Mediums wird auf einen Wert von 6,0 eingestellt und mit 30 ml einer im Schüttelkolben vorgezüchteten Kultur von Lactobacillus delbrueckii subsp. delbrueckii (DSM 20074) beimpft. Die Fermentationstemperatur beträgt 33°C. Die im Verlauf des Prozesses durch die Milchsäurebildung verursachte Acidität im Fermentationsmedium wird mit Hilfe einer automatischen pH-Regulierung durch Zugabe einer 15%igen wäßrigen Piperazinlösung kompensiert. Nach 48 h ist der gesamte vorgelegte Zucker verbraucht, und der Prozeß wird abgebrochen. Die Fermentationslösung enthält nun 154 g D (-)-Milchsäure und 147 g Piperazin. Das entspricht einer Ausbeute an Rohmilchsäure von 90,6%.

Die Fermentationsbrühe wird durch eine Ultrafiltrationseinrichtung geleitet und so von der Biomasse befreit. Das in der klaren Lösung befindliche Piperazonium-(D-)-lactat kann dann mit Hilfe einer gekoppelten Elektrodialyse-Umkehrosmose-Stufe vorgereinigt und aufkonzentriert werden. Die daraus resultierende 50%ige Piperazonium-(D)-lactat-Lösung gelangt in die Eindampfung, wo unter Vakuum der Hauptteil des Wassers entfernt wird. Der Rückstand wird in 2 1 Ethanol (99%) aufgenommen. Aus der filtrierten Lösung erhält man nach dem Einengen 250 g Piperazin-(D)-Lactat als Rohprodukt.

### Beispiel 2:

Eine Lösung von 5 g Pepton, 5 g Hefeextrakt, 2,0 Ammoniumphosphat, 1 g Kaliumhydrogenphosphat in 1 1 Leitungswasser und das gleiche Volumen einer 19%igen Glucoselösung werden getrennt sterilisiert und dann zusammen in einen Rührfermentor gegeben. Der pH-Wert dieses Mediums wird auf einen Wert von 5,0 eingestellt und mit 30 ml einer im Schüttelkolben vorgezüchteten Kultur von Lactobacillus paracasel beimpft. Die Fermentationstemperatur beträgt 33°C. Die im Verlauf des Prozesses durch die Milchsäurebildung verursachte Acidität im Fermentationsmedium wird mit Hilfe einer automatischen pH-Regulierung durch Zugabe einer 15%igen wäßrigen Piperazinlösung kompensiert. Nach 48 h ist der gesamte vorgelegte Zucker verbraucht, und der Prozeß wird abgebrochen. Die Fermentationsbrühe enthält nun 175 g L(+)-Milchsäure und 85 g Piperazin. Die Ausbeute an Rohmilchsäure beträgt 92%. Milchsäure und Piperazin liegen in diesem Fall in einem Molverhältnis von 2:1 vor.
Nach der Aufarbeitung, die analog der in Beispiel 1 beschriebenen Prozedur erfolgt, erhält man 220 g 1,4-Piperazonium-(L,L)-dilactat als Rohprodukt.

### Beispiel 3

Allgemeine Darstellung von racemischem und optisch reinem 1,4-Piperazonium-dilactat und 1,4-(Alkylpiperazonium)-dilactat aus wäßriger Milchsäurelösung Ca. 100 g einer wäßrigen Milchsäurelösung [Anteil an Milchsäure ($R^1$ = H; $R^2$ = $CH_3$) > 70%; enthält u.a. Kondensationsprodukte, wie Lactoylmilchsäure und Polymilchsäure] werden in einem schlanken 1 1-Reaktionsgefäß mit Bodenablaßeinrichtung, Kühlmantel, Rührer und Innenthermometer unter gutem Rühren mit der ca. halben molaren Menge an gepulvertem Piperazin ($R^3$ bis $R^{10}$=H) bzw. Alkylpiperazin ($R^3$ bis $R^{10}$=Alkyl) in Form der freien Amine bzw. ihrer Hydrate versetzt, wobei die Reaktionsrauminnentemperatur 80°C nicht übersteigen soll.

[Die Reaktionswärme kann je nach Ansatzgröße und eingesetztem Piperazin 95°C übersteigen. Unter Umständen kann es sinnvoll sein, den Reaktor nicht zu kühlen, da zum einen auf diese Weise das Wasser zu einem großen Teil entfernt, und zum anderen die Reaktionswärme z.B. zum Erwärmen der Kristallisationsmittel positiv genutzt werden kann. Dies führt jedoch, je nach Ansatz und Reinheit der Einsatzprodukte zu einer mehr oder minder starken $CO_2$-Entwicklung, was wiederum zu Lasten der Ausbeuten geht.]

Nach Beendigung der Zugabe erstarrt die Mischung je nach Wasser- und Polymerengehalt der Ausgangslösungen zu einer festen, wachsartigen oder öligen Masse. Man läßt etwas stehen bis sich die Konsistenz der Mischung nicht mehr sichtbar verändert. Nach Zugabe von ca. 350 ml technischem Ethylenglycolmonobutylether bzw. alternativ einem $C_2$-$C_4$-Ethylenglycolmonoalkyl- oder Ethylenglycoldialkylether) wird langsam auf 70 - 75 °C erwärmt, bis die Lösung klar ist. Bildet sich in der noch heißen Lösung ein Bodensatz, wird dieser entfernt und das noch warme Filtrat dem Reaktor zugeführt und b.B. die gesamte Lösung durch Erwärmen geklärt. Nach Abkühlen tritt sehr schnell Kristallisation ein.

[Ist die Lösung stark dunkel (gelb bis braun) gefärbt, wird die Lösung unter Zugabe von Aktivkohle und/oder Molsieb 4A ca. 20 min bei 80°C gerührt und nach Entfernen der Absorbentien nach AAV weiterverarbeitet. Bei sehr starken Verunreinigungen, vor allem bei öligen dunklen Reaktionsprodukten wird in trockenem Ethanol gelöst, mit Aktivkohle und Molsieb versetzt, 30 min unter Rückfluß erhitzt und über Nacht stehengelassen. Nach Filtrieren der Lösung und destillativer Entfernung des Lösungsmittels wird der Rückstand aus Ethylenglycolmonobutylether oder einem $C_2$-$C_4$-Ethylenglycolmonoalkyl- oder Ethylenglycoldialkylether bei ca. 70°C umkristallisiert.]

Es wird abgesaugt oder filtriert, der Rückstand mit Diethylether oder einem niederen Glycolether gewaschen und nach an sich üblichen Methoden an der Luft, im Luft- oder Inertgasstrom oder im Vakuum über einem wasserabsorbierenden Mittel getrocknet. Es wird aus $C_2$-$C_4$-Ethylenglycolmono- oder Ethylenglycoldialkylether, Dioxan, Essigsäureethylester bzw. Acetonitril umkristallisiert ( *vgl.* Tabelle 1). Die isolierten 1,4-Piperazoniumdilactate schmelzen je nach Reinheit, Trocknungsgrad und optischer Spezifikation in einem Bereich von 96-116°C.

**Beispiel 4**

1,4-Piperazonium-(D,L)-dilactat

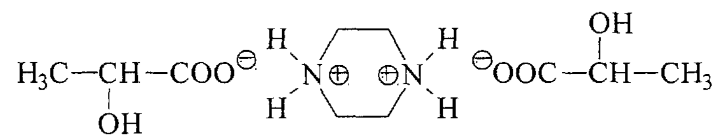

103.50 g 85 %iger D,L-Milchsäure (1.0 mol reine Milchsäure) werden in einem schmalen, zylinderförmigen 31-Reaktionsgefäß (mit Bodenstutzen, Innenthermometer, Rührer ) vorgelegt und unter Rühren mit 43.07 g (0.5 mol) wasserfreiem Piperazin versetzt, wobei die Temperatur sehr schnell auf 98°C steigt und Wasser zu einem Teil abdampft. Man läßt unter Rühren abkühlen, wobei die Mischung bei ca. 55°C zähflüssig (aber noch gut rührbar) wird und bei 40°C zu erstarren beginnt. Die feste wachsartige Masse wird mit 2,3 Liter technischem Acetonitril versetzt und unter Rühren zum Sieden erhitzt. Bleibt nach Beendigung des Rührvorganges ein Bodensatz zurück, so wird dieser über den Reaktorabfluß entfernt, heiß filtriert und das Filtrat der Lösung im Reaktor zugeführt. Man läßt den Reaktor abkühlen, wobei das Piperazin-dilactat auskristallisiert. Man entfernt das Lösungsmittel und wäscht unter Rühren den Kristallbrei mit 100 ml 30-35°C warmen Ethylenglycolmonoethylether. Es wird abgesaugt oder filtriert, mit kaltem Ethylenglycolmonoethylether gewaschen und an der Luft oder im Vakuum getrocknet. Man erhält 124.5 g (94.5 %) luftgetrocknetes 1,4-Piperazonium-(D,L)-dilactat in schönen weißen Nadeln mit einem Schmelzbereich von 109-111°C. Nach Umkristallisieren aus Ethylenglycolmonobutylether bei einer Temperatur von 70°C und Trocknen i. Vak. über $P_4O_{10}$ schmilzt das Lactat im Bereich von 110-111°C.

**Beispiel 5**

1,4-Piperazonium-(D,D)-dilactat

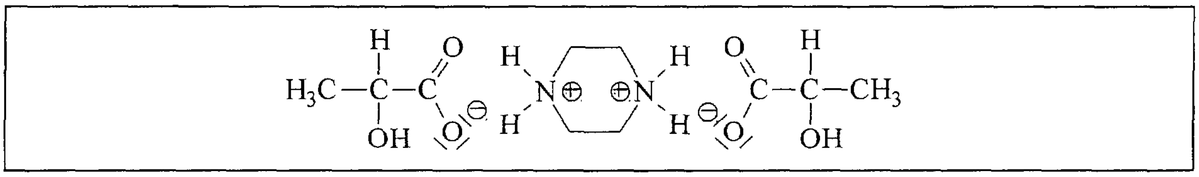

100.0 g 90 %iger wäßriger *D(-)-Milchsäure*-Lösung (Stereochemische Reinheit: 97%) werden in einem schlanken 11-Reaktionsgefäß mit Ablaßeinrichtung (ev. Kühlmantel), Rührer und Innenthermometer eingewogen. Unter gutem Rühren versetzt man die Lösung portionsweise mit 57.0 g (0.5 mol) gepulvertem *Piperazin* (*trans*-Isomerengehalt > 92 %; Fp.: 111-113°C) und zwar so, daß die Temperatur 80°C nicht übersteigt (evt. Kühlen). Nach Beendigung der Zugabe erstarrt die Mischung sehr schnell zu einer wachsartigen weißen Masse (bei T: ca. 45°C). Man läßt etwas stehen und löst die Masse unter Rühren in 350 ml ca. 55°C warmem Ethylenglycolmonobutylether (EGBE). Die Mischung wird unter Rühren auf 70°C erwärmt und nach Entfernen des Bodensatzes stehengelassen, wobei Kristallisation eintritt. Durch Kühlung des Reaktormantels kann die Kristallisation beschleunigt werden. Es wird abgesaugt oder filtriert, mit Diethylether gewaschen und nach üblichen Verfahren an der Luft oder im Vakuum über einer wasserabsorbierenden Substanz getrocknet. Man erhält 115.5 g (78.6 %) farblose bis weiße Nadeln an *Piperazonium-(D,D)-dilactat* mit einem Schmelzpunkt von 108-110°C [ ( $CH_3CN$) Fp.: 111-112°C] und einem spezifischen Drehwinkel $[\alpha]_D^{20}$ von +3,8° (c=10 g in 10 ml Wasser).

Es kann aus Acetonitril, Dioxan, Essigsäureethylester sowie Ethylenglycolmono- und Ethylenglycoldialkylether umkristallisiert werden.

Tabelle 2

| Elementaranalyse: $C_{10}H_{22}N_2O_6$ ( 266.30 ) | | | | |
|---|---|---|---|---|
| | C | H | N | O |
| ber. | 45.10 | 8.33 | 10.52 | 36.05 |
| gef. | 44.96 | 8.45 | 10.46 | — |

**Beispiel 6**

1,4(2,5-Dimethylpiperazonium)-(D,D)-dilactat

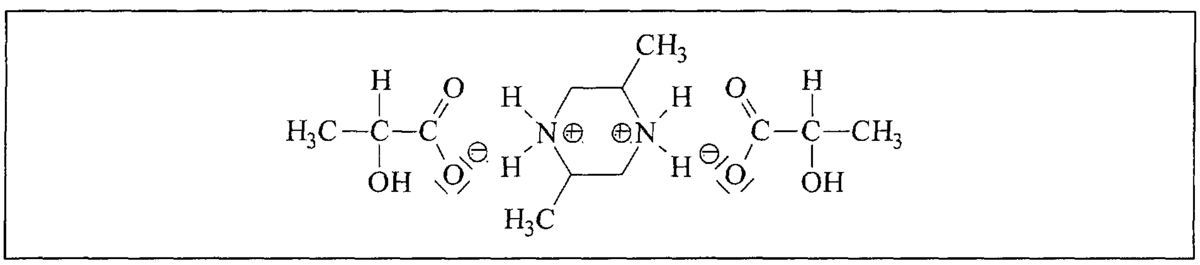

100.0 g 90 %iger wäßriger D(-)-Milchsäure-Lösung (Stereochemische Reinheit: 97%) werden in einem schlanken 11-Reaktionsgefäß mit Ablaßeinrichtung (ev. Kühlmantel), Rührer, und Innenthermometer eingewogen. Unter gutem Rühren versetzt man die Lösung mit 57.0 g gepulvertem 2,5-Dimethylpiperazin (*trans*-Isomerengehalt > 92 %; Fp.: 111-113°C) wobei die Temperatur auf ca. 70°C steigt und rasch zu einer wachsartigen weißen Masse (bei T: ca. 45°C)

erstarrt. Steigt die Innentemperatur über 80°C sollte das Reaktionsgefäß gekühlt werden. Man läßt etwas stehen und löst die Masse unter Rühren in 350 ml ca. 55°C warmem Ethylenglycolmonobutylether (EGBE). Die Mischung wird unter Rühren auf 70°C erwärmt, der Bodensatz entfernt und stehengelassen, wobei Kristallisation eintritt. Durch Kühlung des Reaktormantels kann die Kristallisation beschleunigt werden. Es wird abgesaugt oder filtriert, mit Diethylether gewaschen und nach üblichen Verfahren an der Luft oder im Vakuum über einer wasserabsorbierenden Substanz getrocknet. Man erhält 122 g (83 %) transparente bis weiße Kristalle an luftgetrocknetem *1,4-(2,5-Dimethylpiperazonium)-(D,D)-dilactat* mit einem Schmelzpunkt von 138-140 °C und einem spezifischen Drehwinkel $[\alpha]_D^{20}$ von +7.4° (c=10 g in 10 ml Wasser).
(*vgl*. Tabelle 1)

Tabelle 3

| Elementaranalyse: $C_{12}H_{26}N_2O_6$ ( 294.35 ) | | | | |
|---|---|---|---|---|
| | C | H | N | O |
| ber. | 48.97 | 8.90 | 9.52 | 32.61 |
| gef. | 49.13 | 9.05 | 9.34 | 32.52 |

**Beispiel 7**

1,4-Piperazonium-di-lactat aus dünnen wäßrigen Milchsäurelösungen

**Beispiel 7/1**

1,4-Piperazonium-(L,L)-dilactat aus einer 50 %igen wäßrigen L(+)-Milchsäurelösung

510 g einer ca. 50 %igen wäßrigen *L(+)-Milchsäurelösung* (enthält u.a. Kondensationsprodukte, wie Lactoylmilchsäure und Polymilchsäure und Wasser) werden in einem 2,5 1 Reaktionsgefäß mit Bodenablaßeinrichtung (evt. Kühlmantel), Innenthermometer, Rührer und Wasserabscheidersystem unter Rühren mit 275 g (1.42 mol) pulverisiertem *Piperazin-6-hydrat* versetzt, wobei die Temperatur auf ca. 45°C ansteigt. Nach Zugabe von ca. 650 g technischem Benzen werden unter Rühren und Erwärmen ca. 670 ml Wasser sehr schnell azeotrop am Wasserabscheider abdestilliert. Man engt die Reaktionslösung durch Destillation von Benzen ein (ca. 400-450 ml), bis die Masse zu kristallisieren beginnt. Das Salz erstarrt zu plastischen transparenten Klumpen, das restliche Lösungsmittel wird über das Bodenventil abgenommen. Die Masse wird im Reaktionsgefäß mit 1200 ml Etylenglycolmonobutylether (alternativ, -propylether, -ethylether) versetzt und unter Rühren auf 60°C erwärmt; das Produkt löst sich. Man erwärmt auf 70-75°C bis die Lösung klar ist und entfernt den Bodensatz durch Filtration. Nach Zugabe des Filtrates zur restlichen noch warmen Reaktionslösung läßt man auskristallisieren. Zur Verfeinerung der Kristallisation wird die Lösung noch einmal bis zur Klare erwärmt und ohne Kühlung stehengelassen. Das Kristallisationsmedium wird abgelassen und das Salz mit Diethylether (bzw. einem $C_3$-$C_5$-Diether) gewaschen, über einem Filter oder Fritte abgesaugt, evt. mit Ether nachgewaschen und nach den üblichen Methoden getrocknet. Man erhält 326 g (86,8% bez. auf theor. Einwaage reiner Milchsäure) luftgetrocknetes *1,4-Piperazonium-(L,L)-dilactat* mit einem Schmelzbereich von 104-114°C. Nach Trocknen im Vakuum über $P_4O_{10}$ wird ein Schmelzbereich von 111-113°C und ein spezifischer Drehwinkel $[\alpha]_D^{20}$ von -3,1° erhalten (c=10 g in 10 ml Wasser).

**Beispiel 7/2**

1,4-Piperazonium-(D,L)-dilactat aus einer 10 %igen wäßrigen (D,L)-Milchsäurelösung

1100 g einer ca. 10 %igen wäßrigen *D,L-Milchsäurelösung* (bestehend aus einem nicht optisch aktiven Isomerengemisch von (DL)-2-Hydroxypropionsäure, ihren Kondensationsprodukten, wie Lactoylmilchsäure und Polymilchsäure und Wasser) werden in einem 3 1 Reaktionsgefäß mit Bodenablaßeinrichtung (evt. Kühlmantel), Innenthermometer, Rührer und Wasserabscheidersystem unter Rühren mit 52.6 g (0.61 mol) pulverisiertem *Piperazin* (wasserfrei) versetzt, wobei die Temperatur auf ca. 35°C ansteigt. Nach Zugabe von ca. 900 g technischem Benzen werden unter Rühren und Erwärmen ca. 990 ml Wasser sehr schnell azeotrop am Wasserabscheider abdestilliert und wie unter Beispiel 5/1 aufgearbeitet. Man erhält 126 g (77,6% bez. auf theor. Einwaage reiner Milchsäure) luftgetrocknetes *1,4-Piperazonium-(D,L)-dilactat* mit einem Schmelzbereich von 108-110°C. Nach Trocknen im Vakuum über $P_4O_{10}$ von 110-111°C.

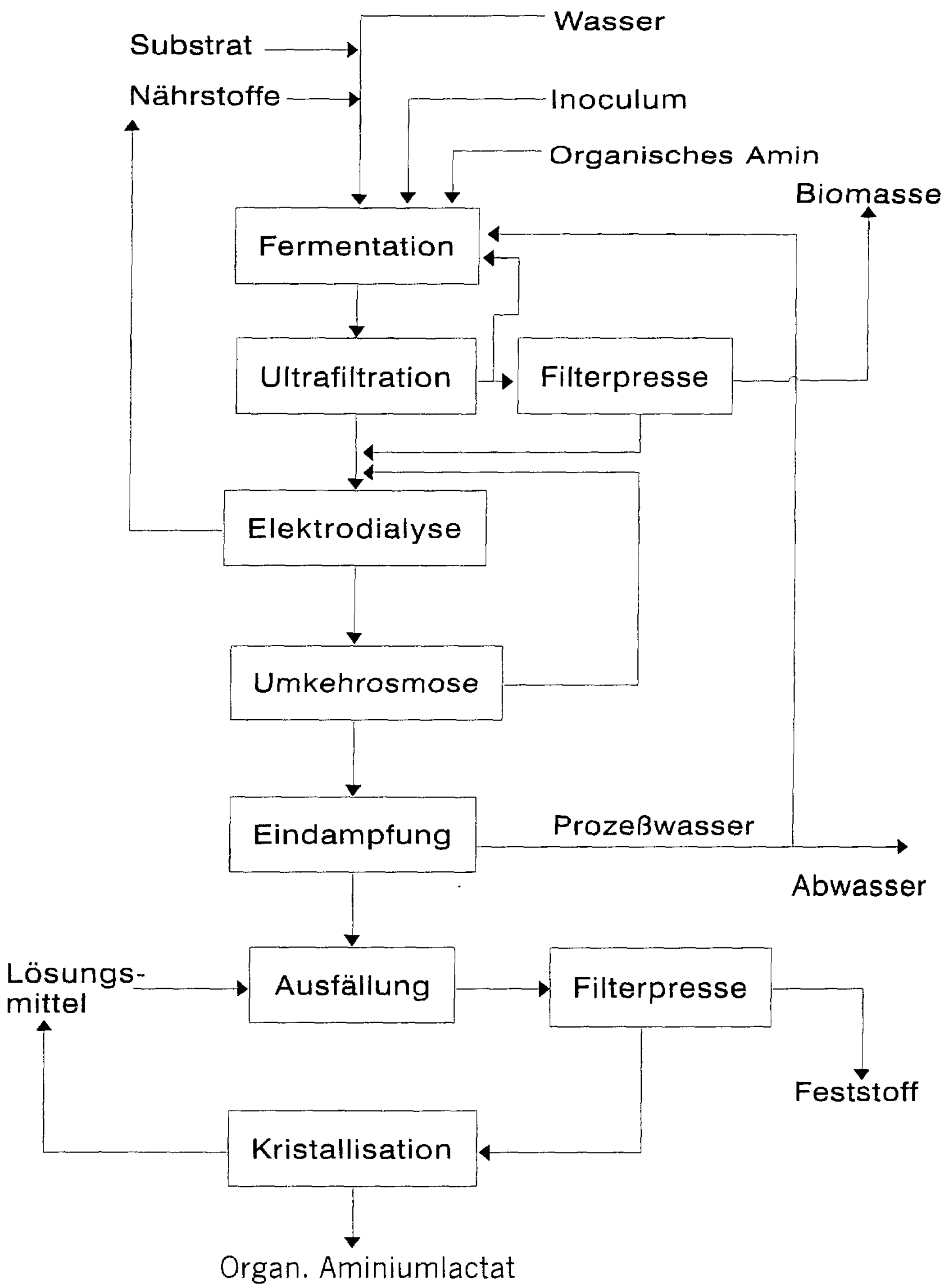


Abb. 2

Prozeßschema des erfindungsgemäßen Fermentationsverfahrens

Tabelle 1

Isolierte Aminiumsalze aus Milchsäure (Auswahl)*

| Verbindung | Hergestellt nach Ausf.-Beispiel | Ausbeute [%] | Schmelzpunkt /- bereich in [°C] | Fp.: 72 h über $P_4O_{10}$ i. Vak. | spez. DW1) $[\alpha]_D^{20}$ |
|---|---|---|---|---|---|
| 1,4-Piperazonium-(L,L)-dilactat | 2 | 82.6 | 112-13 d) | 114-15 | -3.5 a) |
| | *analog* 5 | 88.7 | 111-12 a) 114-15 e) 116 d) | 112-13 a) | -3.5 a) |
| | 7/1 | 86.8 | 104-114 c) | 111-13 a) | -3.1 |
| 1,4-Piperazonium-(D,D)-dilactat | 5 | 78.6 | 108-10 a) 111-112 d) | 109-10 a) | + 5.0 a) |
| 1,4-Piperazonium-(D,L)-dilactat | 4 | 94.5 | 109-111 (waschen mit b) | 110-11 | 0 |
| | 7/2 | 77.6 | 108-110 a) | 110-11 | 0 |
| | | | | | |
| 1,4-(2,5-Dimethylpiperazonium)-(D,D)-dilactat | 6 | 83,3 | 138-140 a) | 139-41 | +7.4 |
| 1,4-(2,5-Dimethylpiperazonium)-(L,L)-dilactat | *analog* 6 | 91,4 | 133-134 a) | 138-40 | -7.2 |
| 1,4-(2,5-Dimethylpiperazonium)-(D,L)-dilactat | *analog* 6 | 90,8 | 151-153 a) | 157-59 a) | 0 |
| | | | | | |
| | | | | | |
| Imidazolium-(L)-lactat | analog 3 | 83,8 | 76-78 (Rohprod); | 81 d) | — |
| 4-Dimethyl-aminopyridinium-(L)-lactat | analog 3 | 85,4 | 97-98 (Rohprod); | 98-99 d) | — |

1) DW-Drehwinkel: Konzentration der gemessenen Salze in Wasser = 1; a) Rohprodukt aus/ bzw. Umkristallisiert aus Ethylenglycolmonobutylether; b) Ethylenglycolmonoethylether; c) Reaktionsmischung in Ethylenglycolmonoethylether; d) Acetonitril; e) Dioxan; f) Dimethylformamid

*die Strukturen der aufgeführten Verbindungen wurden durch Elementaranalyse, IR-, $^1H$-NMR-, und $^{13}C$-NMR-Spektroskopie gesichert.

**Patentansprüche**

1. Verfahren zur Herstellung von organischen Aminiumlactaten oder Diaminiumdilactaten bei der Fermentation von kohlenhydrathaltigen Substraten zur Erzeugung von Milchsäure, **dadurch gekennzeichnet, daß** dem Fermentationsmedium zur Neutralisierung der Milchsäure ein Amin oder Diamin zugesetzt wird, das für die eingesetzten Mikroorganismen nicht toxisch ist und auch deren Aktivität nicht hemmt, so daß am Ende der Fermentation ein pH-Wert des Fermentationsmediums von 4,5 bis 13,5 vorliegt, wobei als Amin oder Diamin eine solche Verbindung eingesetzt wird, die mit Milchsäure stabile und gut kristallisierbare Salze bildet, anschließend das Fermentationsmedium in an sich üblicher Weise von suspendierten Stoffen und einem Teil der gelösten Stoffe befreit wird, das Aminiumlactat oder Diaminiumdilactat im Fermentationsmedium aufkonzentriert und zuletzt in an sich üblicher Weise in kristalliner Form als racemisches oder optisch aktives Salz aus dem Konzentrat gewonnen wird.

2. Verfahren nach Anspruch 1, **dadurch gekennzeichnet, daß** das Amin während der Fermentation zum Konstanthalten des pH-Wertes zudosiert wird und gegebenenfalls die Zudosierung nach Beendigung des Fermentationsprozesses so lange fortgeführt wird, bis der pH-Wert des Fermentationsmediums im Bereich von 4,5 bis 13,5, vorzugsweise im Bereich von 5,0 bis 8,0, liegt.

3. Verfahren nach Anspruch 1, **dadurch gekennzeichnet, daß** das Amin dem Fermentationsmedium oder einem davon abgeleiteten Teilstrom der Produktaufarbeitung zur Neutralisierung der Milchsäure außerhalb des Fermentors zugesetzt wird.

4. Verfahren nach einem der Ansprüche 1-3, **dadurch gekennzeichnet, daß** als Amin oder Diamin ein heterocyclisches sekundäres oder tertiäres Amin oder Diamin oder ein Aminozucker eingesetzt wird.

5. Verfahren nach Anspruch 4, **dadurch gekennzeichnet, daß** Piperazin, Imidazol, Piperidin, Pyrrolidin, 4-Dimethylaminopyridin, D-Glucosamin oder deren für die eingesetzten Mikroorganismen nicht toxischen Derivate, Salze oder Hydrate eingesetzt werden.

6. Verfahren nach einem der Ansprüche 1 bis 5, **dadurch gekennzeichnet, daß** die abgetrennte Biomasse oder ein Teil davon und die in der Elektrodialyse anfallende Fraktion von nichtionischen Medienbestandteilen wieder in die Fermentationsstufe zurückgeführt werden und der abgereicherte Stoffstrom aus der Umkehrosmose zwischen dieser und der Elektrodialyse solange zirkuliert, bis der überwiegende Anteil des organischen Lactates abgetrennt ist.

7. Diaminiumdilactate der allgemeinen Formel V

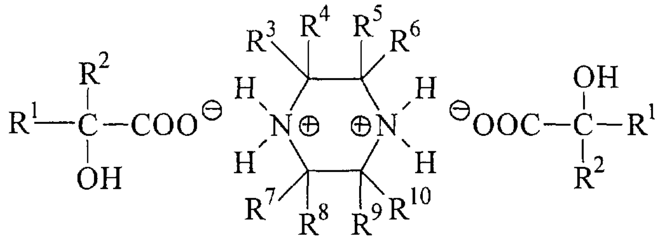

(V)

in der

$R^1$ und $R^2$ unabhängig voneinander Wasserstoff oder $CH_3$ bedeuten,

und

$R^3$ - $R^{10}$ unabhängig voneinander Wasserstoff, verzweigtes oder unverzweigtes $C_1$ - $C_{10}$ -Alkyl, $C_1$ - $C_{10}$ -Alkoxy, Aralkyl mit 1 bis 10 aliphatischen Alkylgruppen zwischen Aromat und Piperazinkörper, Aryl und durch Alkyl-, Alkoxy-,Aryl-, Halogen-, Nitro-, Cyano-, Sulfonyl- Reste substituiertes Aryl, Allyl,

Halogen, Nitril, Isonitril sowie Teile eines ankondensierten aliphatischen, aromatischen oder heterocyclischen Systems bedeuten,

ausgenommen racemisches 1,4-Piperazoniumdilactat.

**8.** Aminiumlactate umfassend D- oder L-Imidazoliumlactat oder racemisches, D- oder L-4-Dimethylaminopyridiniumlactat.

**9.** Verwendung von heterocyclischen sekundären oder tertiären Aminen oder Diaminen oder von Aminozuckern, die mit Milchsäure stabile und gut kristallisierbare Salze bilden, zur Isolierung von Milchsäure aus Fermentationsbrühen biotechnologischer Milchsäurefermentationen unter Gewinnung der entstandenen Aminium- oder Diaminium-Salze.

## Claims

**1.** A method of preparing organic ammonium lactates or diammonium dilactates in the fermentation of carbohydrate-containing substrates to produce lactic acid, **characterized in that** an amine or diamine is added to the fermentation medium in order to neutralize the lactic acid, which amine or diamine neither is toxic for the microorganisms employed, nor inhibits the activity thereof, so that after completed fermentation, a pH value of from 4.5 to 13.5 is present in the fermentation medium, a compound forming stable and well-crystallizable salts with lactic acid being used as amine or diamine, the fermentation medium subsequently is made free of suspended substances and part of the dissolved substances in a *per se* usual manner, the ammonium lactate or diammonium dilactate in the fermentation medium is concentrated and ultimately recovered in a *per se* usual manner from the concentrate in a crystalline form as a racemic or optically active salt.

**2.** The method according to claim 1, **characterized in that** the amine is metered during fermentation to maintain the pH at a constant value, and metering optionally is continued after completion of the fermentation process until the pH value of the fermentation medium is in a range of from 4.5 to 13.5, preferably in a range of from 5.0 to 8.0.

**3.** The method according to claim 1, **characterized in that** in order to neutralize the lactic acid, the amine is added outside the fermenter to the fermentation medium or to a product work-up split flow derived therefrom.

**4.** The method according to any of claims 1-3, **characterized in that** a heterocyclic secondary or tertiary amine or diamine or an amino sugar is used as amine or diamine.

**5.** The method according to claim 4, **characterized in that** piperazine, imidazole, piperidine, pyrrolidine, 4-dimethylaminopyridine, D-glucosamine, or derivatives, salts or hydrates lacking toxicity for the employed microorganisms are used.

**6.** The method according to any of claims 1-5, **characterized in that** the removed biomass or part thereof, as well as the fraction of non-ionic medium components obtained in the electrodialysis are re-fed into the fermentation stage, and the depleted flow of material from the reverse osmosis is circulated between the latter and the electrodialysis until the major portion of the organic lactate is removed.

**7.** Diammonium dilactates of general formula V

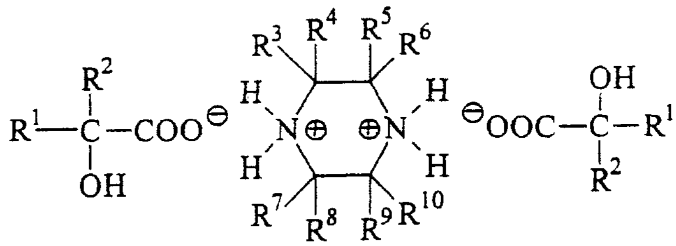

(V)

wherein

$R^1$ and $R^2$ independently represent hydrogen or $CH_3$, and
$R^3$ - $R^{10}$ independently represent hydrogen, branched or non-branched $C_1$-$C_{10}$ alkyl, $C_1$-$C_{10}$ alkoxy, aralkyl having from 1 to 10 aliphatic alkyl groups between the aromatic unit and the piperazine moiety,

aryl, as well as aryl, allyl substituted by alkyl, alkoxy, aryl, halogen, nitro, cyano, sulfonyl residues, halogen, nitrile, isonitrile, as well as parts of a fused aliphatic, aromatic or heterocyclic system,
with the exception of racemic piperazinediium dilactate.

**8.** Ammonium lactates, comprising D- or L-imidazolium lactate, or racemic, D-, or L-4-dimethylaminopyridinium lactate.

**9.** Use of heterocyclic secondary or tertiary amines or diamines or of amino sugars, which form stable and well-crystallizable salts with lactic acid, in the isolation of lactic acid from fermentation broths of biotechnological lactic acid fermentations to recover the ammonium or diammonium salts having formed.

## Revendications

**1.** Procédé de fabrication de lactates d'ammonium ou de dilactates de diammonium organiques par la fermentation de substrats contenant de l'hydrate de carbone et produisant de l'acide lactique, **caractérisé en ce qu'**une amine ou une diamine est ajoutée au milieu de fermentation pour neutraliser l'acide lactique, qui n'est pas toxique pour les microorganismes utilisés et qui ne ralentit pas leur activité, de telle sorte qu'en fin de fermentation on obtient un pH du milieu de fermentation de 4,5 à 13, 5, dans lequel en tant qu'amine ou diamine, on utilise un composé qui forme avec l'acide lactique des sels stables et bien cristallisables, on libère ensuite le milieu de fermentation des matières en suspension et d'une partie des substances dissoutes de manière habituelle, on concentre le lactate d'ammonium ou le dilactate de diammonium dans le milieu de fermentation et on l'extrait enfin du concentré d'une manière habituelle sous forme cristalline en tant que sel racémique ou optiquement actif.

**2.** Procédé d'après la revendication 1, **caractérisé en ce que** l'amine est ajoutée par dosage pendant la fermentation pour maintenir un pH constant et qu'on poursuit éventuellement l'ajout dosé après l'achèvement du procédé de fermentation, jusqu'à ce qu'on obtienne un pH du milieu de fermentation dans une gamme de 4,5 à 13,5, de préférence dans une gamme de 5,0 à 8,0.

**3.** Procédé d'après la revendication 1, **caractérisé en ce que** l'on ajoute l'amine à l'extérieur du fermenteur au milieu de fermentation ou à un flux partiel d'achèvement du produit qui en est détourné pour neutraliser l'acide lactique.

**4.** Procédé d'après une des revendications de 1 à 3, **caractérisé en ce qu'**en tant qu'amine ou diamine, on utilise une amine ou une diamine hétérocyclique secondaire ou tertiaire, ou un sucre aminé.

**5.** Procédé d'après la revendication 4, **caractérisé en ce que** l'on utilise de la pipérazine, de l'imidazole, de la pipéridine, de la pyrrolidine, de la 4-diméthylaminopyridine, du D-glucosamine, ou leur dérivé, leur sel ou leur hydrate non toxique pour les microorganismes utilisés.

**6.** Procédé d'après une des revendications de 1 à 5, **caractérisé en ce que** la biomasse séparée ou une partie d'entre elle et la fraction des composants du milieu non ioniques obtenue par électrodialyse sont ramenées de nouveau vers l'étape de fermentation et que le flux de matière appauvri par osmose inverse circule entre l'électrodialyseur et cet osmoseur jusqu'à ce que la plus grande partie du lactate organique soit séparée.

**7.** Dilactate de diammonium de formule générale V

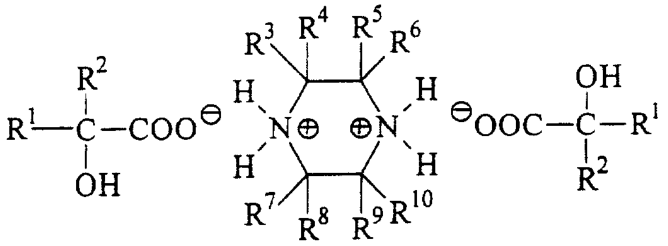

(V)

dans laquelle

$R^1$ et $R^2$ représentent indépendamment l'un de l'autre de l'hydrogène ou $CH_3$, et
$R^3$ à $R^{10}$ représentent indépendamment l'un de l'autre de l'hydrogène, un alkyle en $C_1$-$C_{10}$, un alcoxy en $C_1$-$C_{10}$ ramifié ou non ramifié, un aralkyle comportant de 1 à 10 groupes alkyles aliphatiques entre le noyau aromatique et le corps de pipérazine, un aryle et un aryle, un allyle substitué par des radicaux alkyle, alcoxy, aryle, halogène, nitro, cyano, sulfonyle, un halogène, un nitrile, un isonitrile ainsi que des parties d'un système aliphatique, aromatique ou hétérocyclique condensé,

à l'exception du pipérazinediium dilactate racémique.

**8.** Lactate d'ammonium, comprenant le D- ou le L-imidazolium lactate ou le D-, le L-4-diméthylaminopyridinium lactate ou ce dernier composé racémique.

**9.** Utilisation d'amines ou de diamines hétérocycliques secondaires ou tertiaires, ou de sucres aminés, qui forment avec l'acide lactique des sels stables et bien cristallisables, pour isoler l'acide lactique des bouillons de fermentation des fermentations d'acide lactique biotechnologiques en extrayant les sels d'ammonium ou de diammonium obtenus.